# FASCICULE DE BREVET EUROPEEN

(11) **EP 0 735 042 B1**
(45) Date de publication et mention de la délivrance du brevet: **24.05.2000**
(21) Numéro de dépôt: 96400650.6
(22) Date de dépôt: 27.03.1996
(51) Int. Cl.: C07H 15/04

(54) **Composition de maltitol et son procédé de préparation**
Maltitol Zusammensetzung und Verfahren zu ihrer Herstellung
Maltitol composition and its process of preparation

(30) Priorité: 29.03.1995 FR 9503732; 06.06.1995 US 470461
(43) Date de publication de la demande: 02.10.1996
(73) Titulaire: ROQUETTE FRERES, 62136 Lestrem (FR)
(72) Inventeur: Caboche, Jean-Jacques, 62400 Bethune (FR)
(74) Mandataire: Boulinguiez, Didier

(56) Documents cités:
- EP-A- 0 185 595
- EP-A- 0 189 704
- EP-A- 0 202 165
- EP-A- 0 491 953
- EP-A- 0 561 585
- WO-A-92/16542
- CARBOHYDRATE RESEARCH., vol. 108, 1982, AMSTERDAM NL, pages 163-171, XP002005731 S.OHNO ET AL.: "X-ray Crystal Structure of Maltitol (4-O-alpha-D-Glucopyranosyl-D-Glucitol)"
- BULLETIN OF THE KOREAN CHEMICAL SOCIETY, vol. 10, no. 4, 1989, pages 352-356, XP002005732 J.Y.PARK ET AL.: "The Crystal and Molecular Structure of Maltitol"

## Description

La présente invention concerne une nouvelle composition cristalline de maltitol, d'une très grande pureté et de faible densité. Elle a trait également à un procédé particulier d'obtention de cette composition et aux utilisations de celle-ci dans l'industrie.

Le 4-O-alpha-D-glucopyranosyl-D-glucitol, appelé communément maltitol, est un polyol obtenu industriellement par hydrogénation du maltose. Il présente un grand intérêt en raison du fait qu'il est plus stable chimiquement et moins calorique que le saccharose, tout en possédant avantageusement des propriétés organoleptiques très voisines de celles de ce sucre. De plus, le maltitol possède la particularité de n'être pas cariogène, ce qui lui ouvre et lui a déjà ouvert de multiples applications dans l'industrie, notamment dans les industries alimentaires et pharmaceutiques.

Pendant très longtemps, le maltitol n'a été présenté que sous forme de sirops de faible richesse. Ce polyol est par exemple le composé majoritaire présent dans les sirops LYCASIN® 80/55 et MALTISORB® 75/75 commercialisés depuis près de vingt ans par la demanderesse. Les teneurs en maltitol de ces sirops ne dépassent jamais 78 % de leur matière sèche.

Puis, le maltitol a été commercialisé sous forme de poudres amorphes et impures. On a ainsi souvent séché par atomisation des solutions de maltitol. Si l'on se réfère à la littérature, cette technique a toujours été considérée comme particulièrement difficile à mettre en oeuvre en raison d'un collage important dans les tours d'atomisation mais aussi en raison du caractère très hygroscopique des poudres ainsi obtenues. De nombreux brevets témoignent d'un travail important visant à remédier à ces problèmes. On peut citer par exemple :
- les brevets GB 1 383 724, JP 49-87619 et US 4 248 895, dans lesquels il est proposé d'ajouter avant atomisation aux solutions de maltitol diverses substances telles que des alginates, celluloses, amidons modifiés, polyvinylpyrrolidone, polymères hydrophiles, protéines ou extraits protéiques, de façon à réduire le collage dans les tours d'atomisation.
- les brevets JP 50-59312 et JP 51-113813, dans lesquels sont décrites des méthodes d'atomisation de compositions anhydres de maltitol fondu.
- les brevets JP 49-110620, US 3 918 986, US 3 915 736, JP 50-129769 et JP 48-61665, dans lesquels sont données des méthodes visant à réduire l'hygroscopicité des poudres anhydres de maltitol, soit par ajout de substances antimottantes, soit par enrobage des poudres de maltitol par des saccharides, des polyols ou des matières grasses, ou soit encore par granulation humide.

Ce n'est que vers 1980 que l'on a réussi pour la première fois à produire des cristaux de maltitol. Auparavant, ce polyol avait toujours été considéré comme un produit non cristallisable. Ce postulat erroné, resté longtemps ancré dans les esprits, trouve en réalité son origine dans le fait que la cristallisation du maltitol à partir d'une solution sursaturée n'est pas aussi spontanée que dans le cas d'autres polyols comme le mannitol, l'érythritol ou l'isomalt par exemple. Certaines caractéristiques propres au maltitol, comme en particulier sa viscosité et sa solubilité, seraient à l'origine des difficultés constatées.

La seule forme cristalline connue à ce jour pour le maltitol est la forme anhydre, décrite dans le brevet US 4 408 041 de la société HAYASHIBARA. Pour connaître les caractéristiques de cette forme cristalline, on pourra au besoin se référer à ce brevet déposé en 1981, ou à l'article "X-ray crystal structure of maltitol (4-O-alpha-D-glucopyranosyl-D-glucitol)" de SHOICHI OHNO et al., paru dans "Carbohydrate Research, 108(1982), 163-171. Quelques années plus tard sont apparues sur le marché les premières poudres pseudo-cristallines de maltitol. Celles-ci furent, et sont toujours, pour certaines d'entre elles, préparées par une technique dite de "massé", consistant à faire prendre en masse une solution déshydratée de maltitol présentant une richesse pouvant atteindre au mieux 90 %, par ajout d'une amorce composée de cristaux de sucres ou de polyols. Un tel procédé est par exemple décrit dans les documents JP 57-47680 et JP 58-158145.

Il a également été proposé dans le brevet US 4 408 401 cité plus haut, de préparer des mélanges cristallins pulvérulents, appelés "total sugar", par atomisation de solutions pré-cristallisées ou masses cuites. Celles-ci sont obtenues par refroidissement très lent d'une solution aqueuse sursaturée en maltitol, contenant par ailleurs de larges quantités d'autres polyols tels que sorbitol, maltotriitol et maltotétraitol et d'autres polyols de degré de polymérisation supérieur.

Par ce refroidissement très lent et par ajout d'une amorce cristalline de maltitol, on fait apparaître et croître dans la solution des cristaux de maltitol. Lorsque 25 à 60 % du maltitol de cette solution aqueuse est cristallisé, on procède alors à une atomisation à une température très basse, c'est-à-dire comme indiqué, à une température comprise entre 60 et 100°C pour ne pas faire disparaitre les cristaux que l'on a volontairement générés. De ce fait, le "total sugar" obtenu contient 25 à 60 % de maltitol cristallisé sous forme de cristaux tout à fait identiques à ceux obtenus par cristallisation dans l'eau. On verra plus loin les inconvénients dus à la présence de tels cristaux pour certaines applications particulières.

Par ailleurs, ce "total sugar" est loin d'être suffisamment cristallin puisqu'il est indiqué qu'il nécessite, si l'on se réfère à la description et notamment à l'exemple 4, d'être séché davantage, pendant 40 minutes environ, mais aussi d'être vieilli pendant 10 heures. On comprend que ce procédé, très dispendieux en temps, n'ait semble-t-il jamais connu le moindre développement et la moindre attention.

Un pas décisif dans la mise au point de poudres cristallines de maltitol de très haute richesse a été franchi, grâce aux travaux de la demanderesse, par la mise au point de nouveaux procédés de fabrication basés sur l'emploi de techniques de fractionnement par chromatographie continue. Ces procédés, objets des brevets EP 0 185 595 et EP 0 189 704, permettent d'obtenir à un coût compétitif des poudres d'une pureté atteignant 99 %, par la simple cristallisation dans l'eau du maltitol présent dans une fraction chromatographique particulièrement riche en ce polyol. Une telle poudre cristalline est par exemple commercialisée depuis plusieurs années par la demanderesse sous l'appellation MALTISORB® cristallisé.

Les techniques dites de "massé" d'une part et de cristallisation dans l'eau d'autre part, sont aujourd'hui quasiment les seuls procédés employés industriellement. Les produits ainsi obtenus, dont la cristallinité est très variable, conviennent particulièrement bien à certaines applications comme celles du chewing-gum ou du chocolat.

En revanche, il est d'autres applications où ces produits ne sont pas totalement satisfaisants. C'est le cas par exemple lorsque l'on souhaite utiliser du maltitol pour remplacer le saccharose ou le lactose dans les formes sèches pharmaceutiques telles que les gélules, les médicaments du type poudres à dissoudre, les comprimés et les préparations nutritives pulvérulentes à diluer. C'est également le cas lorsque l'on souhaite réaliser le même genre de substitution dans les aliments sucrés tels que les boissons en poudre, les entremets, les préparations pour gâteaux ou les poudres chocolatées ou vanillées pour petit déjeuner.

On constate pour ces applications particulières, aussi bien pour les poudres pseudo-cristallines de maltitol obtenues par la technique de "massé" que pour les poudres cristallines de maltitol contenant des cristaux obtenus par cristallisation de maltitol dans l'eau, que celles-ci présentent un ou plusieurs défauts comme en particulier ceux de s'écouler difficilement, d'être sujettes à une prise en masse ou à un mottage, de ne se dissoudre que très lentement dans l'eau, d'être de mauvais excipients pour compression ou de ne pas satisfaire aux critères d'identification et de pureté imposés par différentes pharmacopées.

Il a pourtant déjà été proposé, dans le cas du maltitol, d'améliorer par extrusion son aptitude à la compression. Une telle méthode est par exemple décrite dans le brevet EP 0 220 103 dont la demanderesse est titulaire. Elle n'est pas idéale en raison du fait qu'elle ne permet malheureusement pas d'améliorer l'ensemble des défauts constatés plus haut pour les produits du marché.

Désireuse d'améliorer l'état de la technique, la demanderesse a donc cherché à mettre au point une composition de maltitol n'ayant pas les défauts d'écoulement, de mottage, de dissolution, ou de compression que présentent les poudres de maltitol connues. Certes, on aurait pu penser que le besoin identifié puisse être satisfait par d'autres polyols. Or, on constate qu'il n'en est rien car aucun d'entre eux ne possède des caractéristiques de solubilité, d'hygroscopicité, de saveur sucrée et de fusion aussi proches du saccharose que le maltitol.

Il est du mérite de la demanderesse d'avoir réussi, contre toute attente, et après avoir mené une recherche approfondie sur le sujet, à préparer une composition cristalline de maltitol ne présentant pas les défauts relevés pour les poudres de maltitol connues. Elle a mis en évidence que, de manière surprenante et inattendue, une telle composition cristalline pouvait être préparée dans des conditions particulières à partir d'un sirop présentant une richesse en maltitol supérieure à 92 %, par un procédé s'apparentant à une atomisation, bien que cette technique n'ait jamais permis dans le passé d'obtenir directement une cristallisation du maltitol . Il faut noter, en outre , que la technique d'atomisation était tombée en totale désuétude dans le cas du maltitol, depuis la mise en évidence de la possibilité de faire cristalliser ce polyol à partir d'une solution sursaturée et l'avènement de procédés jugés très performants basés sur ce principe.

L'invention a trait par conséquent, en premier lieu, à une composition cristalline de maltitol, présentant essentiellement une structure poreuse et alvéolée, une richesse en maltitol supérieure ou égale à 92 %, et une densité apparente comprise entre 100 et 700 g/l.

La notion de richesse doit être entendue, dans le cas de la présente invention, comme correspondant au pourcentage de maltitol exprimé en poids sec/sec par rapport à l'ensemble des carbohydrates présents dans la composition cristalline de maltitol. Ces carbohydrates peuvent être des sucres tels qu'en particulier le D-glucose, le maltose, le maltotriose et le maltotétraose et des polyols issus de l'hydrogénation de ces sucres. Habituellement, cette richesse est mesurée par chromatographie liquide haute performance.

La première caractéristique essentielle de la composition de maltitol tient au fait qu'elle est cristallisée, ce qui lui confère une très haute stabilité vis-à-vis de l'humidité. Elle a par conséquent une faible tendance à prendre en masse ou à motter. Ainsi, son usage est aisé et il n'est pas impératif de prendre des dispositions draconiennes pour prévenir ce risque.

La cristallinité de la composition conforme à l'invention peut être évaluée par calorimétrie thermique différentielle. Celle-ci est directement proportionnelle à son enthalpie de fusion, laquelle est de préférence supérieure à 130 J/g, plus préférentiellement supérieure à 145 J/g et encore plus préférentiellement supérieure à 155 J/g.

Il a été constaté de manière surprenante et inattendue que la composition conforme à l'invention possède une cristallinité toujours nettement supérieure à un maltitol massé, une cristallinité en général supérieure à un maltitol extrudé de richesse en maltitol équivalente et une critallinité en général très légèrement inférieure à un maltitol cristallisé dans l'eau de richesse en maltitol équivalente. C'est ainsi qu'il est courant que la composition conforme à l'invention présente une enthalpie de fusion comprise entre 160 et 164 J/g alors que cette enthalpie de fusion est d'ordinaire comprise entre 80 et 120 J/g pour un maltitol massé comme par exemple dans le cas de la poudre MALBIT® CR, est comprise entre 130 et 145 J/g pour un maltitol extrudé et est comprise entre 163 et 167 J/g pour un maltitol cristallisé dans l'eau tel que le produit MALTISORB® cristallisé, commercialisé par la demanderesse.

Il a été également remarqué que la composition conforme à l'invention présente une température de fusion comprise entre 148 et 150°C, en général voisine de 149°C. Cette température tend à être légèrement plus faible que celle d'un maltitol cristallisé dans l'eau de richesse équivalente en maltitol.

Selon une seconde caractéristique essentielle, la composition cristalline de maltitol conforme à l'invention possède une richesse en maltitol au moins égale à 92 %. On préfère, afin qu'elle puisse cristalliser directement et plus parfaitement, qu'elle présente une richesse en maltitol supérieure ou égale à 95 %, et mieux encore supérieure ou égale à 98 %. L'idéal est d'atteindre une richesse voisine ou supérieure à 99 %.

Par ailleurs, on préfère également que la composition conforme à l'invention ne contienne qu'une faible teneur en polyols autres que le maltitol, ces polyols pouvant être en particulier du sorbitol, du xylitol, du mannitol, de l'iditol, de l'arabitol, du maltotriitol ou du maltotétraitol. La teneur en ces polyols est de préférence inférieure à 5 % et mieux encore inférieure à 2 % par rapport à la matière sèche de la composition. Il a été constaté en effet que leur présence altère de façon significative la cristallinité de la composition conforme à l'invention. Ceci n'est pas le cas, ou l'est dans une bien moindre mesure, lorsque la composition contient certaines autres substances. Ceci explique que la composition cristalline de maltitol puisse contenir sans inconvénient de telles substances en quantité plus ou moins grande, en fonction de l'usage qui lui est réservé.

Parmi les substances susceptibles d'entrer sans problème majeur dans la composition cristalline de maltitol, on peut citer par exemple les édulcorants intenses, les colorants, les parfums, les arômes, les vitamines, les minéraux, les principes actifs pharmaceutiques ou vétérinaires, les esters d'acides gras, les acides organiques ou minéraux et leurs sels, les matières protéiques comme les protéines, les acides aminés et les enzymes.

Selon une troisième caractéristique essentielle, la composition cristalline de maltitol conforme à l'invention présente d'ordinaire une densité plus faible que celles des poudres de maltitol connues. Cette densité peut être mesurée par exemple en utilisant un appareil commercialisé par la société HOSOKAWA sous la marque "Powder Tester" en appliquant la méthode recommandée pour mesurer une densité apparente. Dans ces conditions, la composition conforme à l'invention présente une densité apparente comprise entre environ 100 et environ 700 g/l, de préférence entre 200 et 670 g/l et plus préférentiellement entre 300 et 650 g/l. De façon courante, sa densité apparente est comprise entre 400 et 650 g/l.

La densité faible de la composition conforme à l'invention est due à sa structure particulière, qui la distingue nettement des poudres de maltitol connues. En effet, par observation au microscope, on peut constater que sa structure est essentiellement poreuse et alvéolée. De plus, les particules composant la composition conforme à l'invention sont essentiellement sphériques, dépourvues d'arêtes vives et composées d'une multitude de micro-particules cristallines agglomérées entre elles. Cette structure se différencie de façon nette de celle d'un maltitol cristallisé dans l'eau et d'un maltitol extrudé, qui sont constitués de particules cubiques ou parallépipédiques très anguleuses, ou encore d'un maltitol massé, présentant une structure très dense comportant des particules faiblement biréfringentes en lumière polarisée.

C'est ainsi que la composition cristalline de maltitol conforme à l'invention est quasiment dépourvue de particules présentant des caractéristiques de forme et d'aspect similaires à celles retrouvées dans les poudres de maltitol cristallisées dans l'eau, extrudés ou massés.

La composition cristalline conforme à l'invention possède en général une surface spécifique inférieure à 0,2 m²/g.

De plus, la demanderesse a constaté, en mesurant la porosité au mercure sur des coupes granulométriques de 160 à 250 microns, que contrairement à une poudre de maltitol cristallisé dans l'eau, la composition conforme à l'invention est constituée de particules possédant des pores ouverts de taille comprise entre 1 et 10 microns. Le volume de ces pores représente en général 0,01 à 0,03 cm³/g, ce qui est plus faible que les volumes ordinaires pour des poudres de maltitol massé ou extrudé.

La teneur en eau, déterminée par étuvage à 130°C pendant 2 heures, de la composition cristalline de maltitol conforme à l'invention est de préférence inférieure à 2 % et plus préférentiellement encore inférieure à 1 %. Généralement, cette teneur est même inférieure à 0,5 %, voire à 0,35 %.

En ce qui concerne les caractéristiques fonctionnelles de la composition cristalline de maltitol conforme à l'invention, la demanderesse a évalué son aptitude à l'écoulement en utilisant l'appareil commercialisé par la société HOSOKAWA. Cet appareil permet de mesurer dans des conditions standardisées et reproductibles, l'aptitude à l'écoulement d'une poudre et de calculer une note d'écoulement appelée également indice de Carr. La composition conforme à l'invention présente une note d'écoulement excellente, comprise entre 70 et 90. Cette valeur est de préférence comprise entre 75 et 90 et plus préférentiellement encore entre 80 et 90. Cette valeur est très voisine de celles de poudres de maltitol de l'art antérieur obtenues par extrusion de cristaux cristallisés dans l'eau.

Par ailleurs, l'aptitude à l'écoulement de la composition selon l'invention est d'ordinaire nettement supérieure à celles des poudres de maltitol obtenues par simple cristallisation dans l'eau ou par la technique de "massé".

On peut penser que l'excellente aptitude à l'écoulement de la composition conforme à l'invention s'explique par la combinaison de plusieurs de ses caractéristiques physico-chimiques, à savoir en particulier l'absence de charges électrostatiques importantes à la surface des particules la constituant, sa richesse en maltitol, sa faible hygroscopicité et enfin la forme caractéristique des particules la constituant. Cette excellente aptitude à l'écoulement est avantageuse car elle rend très aisée le remplissage et la vidange de trémies, de récipients ou d'autres contenants tels que sachets ou gélules par exemple.

Une seconde propriété fonctionnelle essentielle de la composition cristalline de maltitol conforme à l'invention est celle de se dissoudre très rapidement dans l'eau. Pour mesurer cette vitesse de dissolution, on procède selon un test A, qui consiste à introduire dans 150 grammes d'eau déminéralisée et dégazée, maintenue à 20°C et soumise à une agitation à 200 t/minute dans un bêcher de forme basse de 250 ml, 5 grammes exactement d'une coupe granulométrique de 200 à 315 microns du produit à tester. Le temps de dissolution correspond au temps nécessaire, après introduction du produit, pour obtenir une parfaite limpidité visuelle de la préparation. Dans ces conditions, la composition conforme à l'invention possède en général une vitesse de dissolution inférieure à 30 secondes et de préférence inférieure à 26 secondes et plus préférentiellement encore inférieure à 20 secondes. Ces temps sont généralement inférieurs à ceux obtenus avec toutes les poudres de maltitol actuellement commercialisées. On comprend que cette faculté de dissolution rapide soit un avantage indéniable, par exemple dans la fabrication de produits alimentaires ou pharmaceutiques à dissoudre avant leur ingestion.

La composition cristalline de maltitol conforme à l'invention possède également d'autres caractéristiques avantageuses. On peut citer sa très bonne aptitude à être comprimée pour préparer des tablettes à mâcher ou à sucer et sa très bonne aptitude à être mélangée à d'autres produits.

La composition cristalline de maltitol conforme à l'invention est susceptible d'être obtenue en procédant à la pulvérisation d'un sirop relativement riche en maltitol respectivement à la quantité de carbohydrates présents dans ce sirop, sur un lit pulvérulent en mouvement de particules de maltitol cristallisé de pureté au moins égale à celle du sirop. Il a été constaté que la richesse en maltitol du sirop doit être supérieure ou égale à 92 % afin que la cristallisation du maltitol puisse s'opérer en un temps suffisamment bref et de façon importante.

Ce sirop de maltitol est généralement une solution totalement limpide de maltitol ou bien une solution légèrement opaque en raison de la présence éventuelle, mais non désirée, de fins cristaux de maltitol.

La composition cristalline de maltitol peut en particulier être obtenue en mettant en oeuvre le procédé comportant les étapes suivantes :
- préparation d'un sirop de maltitol ayant une matière sèche d'au moins 50 % et présentant une richesse en maltitol supérieure ou égale à 92 %,
- pulvérisation fine de ce sirop sur un lit pulvérulent de particules en mouvement de maltitol cristallisé de richesse au moins égale à celle du sirop ; ce lit ayant une température comprise entre 60 et 110°C; la masse du lit représentant constamment au moins 2 fois la masse du sirop pulvérisé,
- séchage du lit pulvérulent et du sirop afin d'obtenir la composition cristalline de maltitol,
- maturation éventuelle de la composition cristalline de maltitol jusqu'à ce qu'elle présente une cristallinité suffisante et de préférence une enthalpie de fusion supérieure ou égale à 130 J/g,
- recyclage éventuel partiel de la composition cristalline de maltitol afin qu'elle constitue un nouveau lit pulvérulent de maltitol cristallisé.

Contrairement à ce que l'on aurait pu penser, cette technique permet d'obtenir une composition cristalline de maltitol de faible densité et dont la vitesse de dissolution dans l'eau est rapide. Ces propriétés peuvent être ajustées en modifiant la richesse en maltitol du sirop, la matière sèche du sirop, la finesse de la pulvérisation, la nature des particules de maltitol cristallisé constituant le lit pulvérulent, le moyen de mise en mouvement de ces particules, la température du lit, la température de séchage, et les masses respectives du lit et de sirop pulvérisé.

En ce qui concerne la richesse en maltitol du sirop, on préfère qu'elle soit supérieure ou égale à 95 %, mieux encore supérieure ou égale à 98 %, l'idéal étant de choisir une richesse voisine ou supérieure à 99 %.

Il est préférable d'éviter une pulvérisation grossière du sirop, auquel cas on observe un collage, une mauvaise cristallisation du maltitol et une augmentation trop forte de densité, ce qui n'est pas recherché. Aussi, afin que la composition cristalline de maltitol présente les propriétés spécifiques décrites plus haut, il convient de retenir un matériel permettant de former à partir du sirop de très fines gouttelettes, voire un brouillard.

En ce qui concerne la nature des particules du maltitol constituant le lit pulvérulent, on préfère qu'elles présentent également une grande richesse en maltitol, toujours au moins égale à celle du sirop employé. Pour obtenir un bon résultat, il est aussi préférable que ce lit soit également assez peu dense, c'est-à-dire présente une densité inférieure à 700 g/l et mieux encore inférieure à 650 g/l. L'idéal est de retenir pour ce lit des particules de maltitol présentant l'ensemble des caractéristiques de la composition cristalline de maltitol conforme à l'invention. Ceci peut être obtenu en réalisant un recyclage partiel de la composition conforme à l'invention, laquelle joue alors le rôle de lit pulvérulent de maltitol cristallisé. Il est très avantageux de procéder de la sorte mais alors il est préférable de broyer ou de tamiser la composition conforme à l'invention pour ne retenir que les particules de taille inférieure à 150 microns et mieux encore de taille inférieure à 90 microns.

La mise en mouvement des particules constituant le lit pulvérulent peut être obtenue mécaniquement, ou par soufflage d'air. Cette dernière possibilité est préférée car il est facile en choisissant la température de l'air, d'ajuster la température du lit à une valeur comprise entre 60 et 110°C, et en régulant les débits d'air, d'ajuster les propriétés de la composition cristalline de maltitol.

Généralement, on préfère que la température de ce lit soit maintenue entre 65 et 90°C, l'idéal étant de se situer entre 70 et 85°C. On préfère également que la masse du lit pulvérulent représente constamment 3 fois ou mieux 5 fois la masse de sirop pulvérisé. Lorsqu'on procède à un recyclage partiel de la composition selon l'invention afin qu'elle joue le rôle de lit pulvérulent, il suffit d'ajuster le débit d'entrée en sirop pour qu'il ne représente qu'au plus 25 %, ou mieux au plus 17 % du débit d'entrée en composition recyclée.

Le séchage du lit pulvérulent sur lequel a été pulvérisé le sirop doit être conduit de façon à obtenir une teneur en eau finale ne dépassant pas 2 %, de préférence 1 % et plus préférentiellement 0,5 % de la composition.

La demanderesse a démontré que l'on pouvait avantageusement fabriquer en continu la composition cristalline de maltitol, par exemple en utilisant une tour d'atomisation de type M.S.D. de la société NIRO-ATOMIZER laquelle permet grâce à sa conception de reproduire toutes les étapes essentielles du procédé conforme à l'invention.

Ce matériel permet, en effet, de pulvériser très finement à l'aide de la buse qu'il comporte, un sirop ayant une température comprise entre 50 et 100°C et une matière sèche comprise entre 55 et 85 %, sur un lit de particules de maltitol, mis et maintenu en mouvement avec de l'air. De plus, ce matériel permet simultanément d'opérer à un séchage par de l'air chaud. On peut choisir avantageusement une température d'entrée d'air comprise entre 160 et 300°C et des débits en matière entrantes tels que la température de l'air sortant de la tour soit comprise entre 60 et 130°C et mieux encore entre 70 et 90°C. Ce matériel permet également de procéder éventuellement à un recyclage partiel de la composition cristalline de maltitol et de la disperser très finement dans le haut de la tour, autour de la buse de pulvérisation du sirop.

La composition cristalline de maltitol obtenue selon le procédé conforme à l'invention peut au besoin être par la suite granulée, de façon à modifier sa granulométrie. Cette granulation peut être réalisée à l'eau, à la vapeur, ou à l'aide d'un sirop contenant de préférence du maltitol.

La composition cristalline de maltitol conforme à l'invention peut avantageusement être employée en tant qu'agent édulcorant, agent de charge ou de texture, excipient ou support d'additifs divers. Elle est particulièrement recommandée, en raison de ses propriétés spécifiques, à la fabrication de comprimés et de poudres à dissoudre dans les domaines alimentaire et pharmaceutique. Toutefois, rien n'empêche de l'utiliser à une tout autre fin, comme par exemple pour formuler des chewing-gums, des sirops ou des confiseries.

L'invention sera encore mieux comprise à l'aide de l'exemple qui suit, lequel ne se veut pas limitatif et fait seulement état de certains modes de réalisation et de certaines propriétés avantageuses de la composition cristalline de maltitol selon l'invention.

### Exemple 1 : Préparation de compositions cristallines de maltitol selon l'invention et comparaison aux produits de l'art antérieur.

On prépare une solution de maltitol à 75 % de matière sèche par dissolution de cristaux de maltitol présentant une richesse en maltitol de 99,8 %. Cette solution est amenée à 80°C puis maintenue à cette température.

On procède à l'atomisation de cette solution en utilisant une tour de type M.S.D. de la société NIRO ATOMIZER. Pour cela on introduit au préalable dans la tour environ 100 kg de poudre de maltitol cristallisé dans l'eau de fine granulométrie. On retient pour cela la poudre cristalline MALTISORB® P 90 commercialisée par la demanderesse. Celle-ci joue le rôle de lit pulvérulent de maltitol cristallisé. Cette poudre est mise en mouvement par fluidisation avec de l'air à 40°-90°C et par recyclage en haut de la tour après passage sur un broyeur produisant des particules de maltitol cristallisé de taille inférieure à 90 microns.

On procède ensuite à la pulvérisation fine du sirop sur le lit pulvérulent de particules en mouvement de maltitol cristallisé, en ajustant les débits des matières entrantes dans la tour de façon à ce que la quantité de ce sirop pulvérisé ne représente pas plus de 25 % de la quantité recyclée de maltitol pulvérulent. On choisit une température d'air de séchage à l'entrée supérieure de la tour comprise entre 165 et 225°C. La température de l'air sortant de la tour est comprise entre 70 et 90°C.

Dans ces conditions, on constate après 7 heures de fonctionnement de la tour, que la composition de maltitol sortant de celle-ci est quasiment dépourvue de particules présentant des caractéristiques de forme et d'aspect, similaires à celles retrouvées dans la poudre MALTISORB® P 90. En effet, la composition est essentiellement poreuse et alvéolée et est constituée de particules essentiellement sphériques, dépourvues d'arêtes vives et composées d'une multitude de micro-particules cristallines agglomérées entre elles. Cette composition conforme à l'invention est appelée Il. Ses principales caractéristiques sont données dans le tableau ci-après.

On procède également à l'atomisation d'une solution de maltitol moins riche en maltitol, en procédant exactement comme indiqué plus haut. Cette solution contient 95,8 % de maltitol et 2,9 % d'autres polyols. Après 7 heures de fonctionnement de la tour, on constate que le produit sortant est bien cristallisé et présente toutes les caractéristiques de la composition cristalline de maltitol conforme à l'invention. Ce produit est appelé 12. On note que cette composition 12, malgré sa richesse assez faible en maltitol, a cristallisé en un temps relativement bref et de façon suffisamment complète, et cela de façon surprenante, sans avoir eu nullement besoin de réaliser un séchage complémentaire et un vieillissement par soufflage d'air pendant une à vingt heures, comme recommandé dans le brevet US 4.408.041.

On compare les compositions I1 et I2 conformes à l'invention à différentes poudres de maltitol de l'art antérieur, c'est-à-dire :
- une poudre cristalline contenant des cristaux de maltitol obtenus par cristallisation dans l'eau (MALTISORB® P 200);
- une poudre obtenue selon la technique dite de "massé" (AMALTY® MR de la société TOWA CHEMICAL);
- et une poudre de maltitol, extrudée selon les conditions données dans le brevet EP 0 220 103.

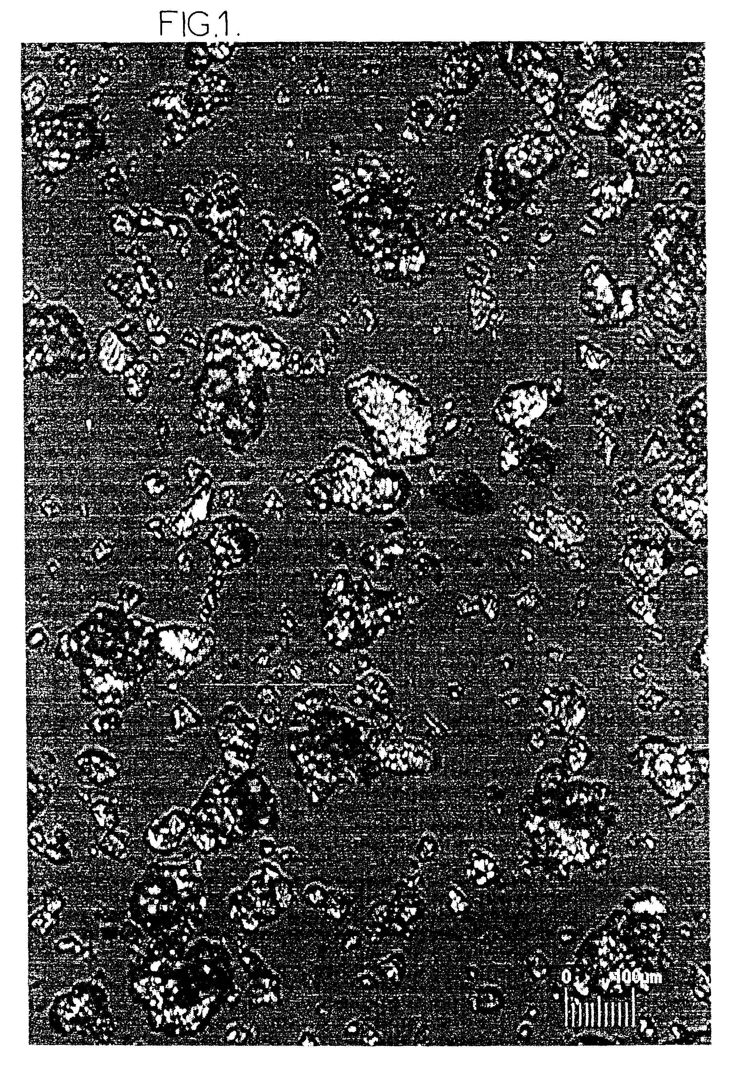

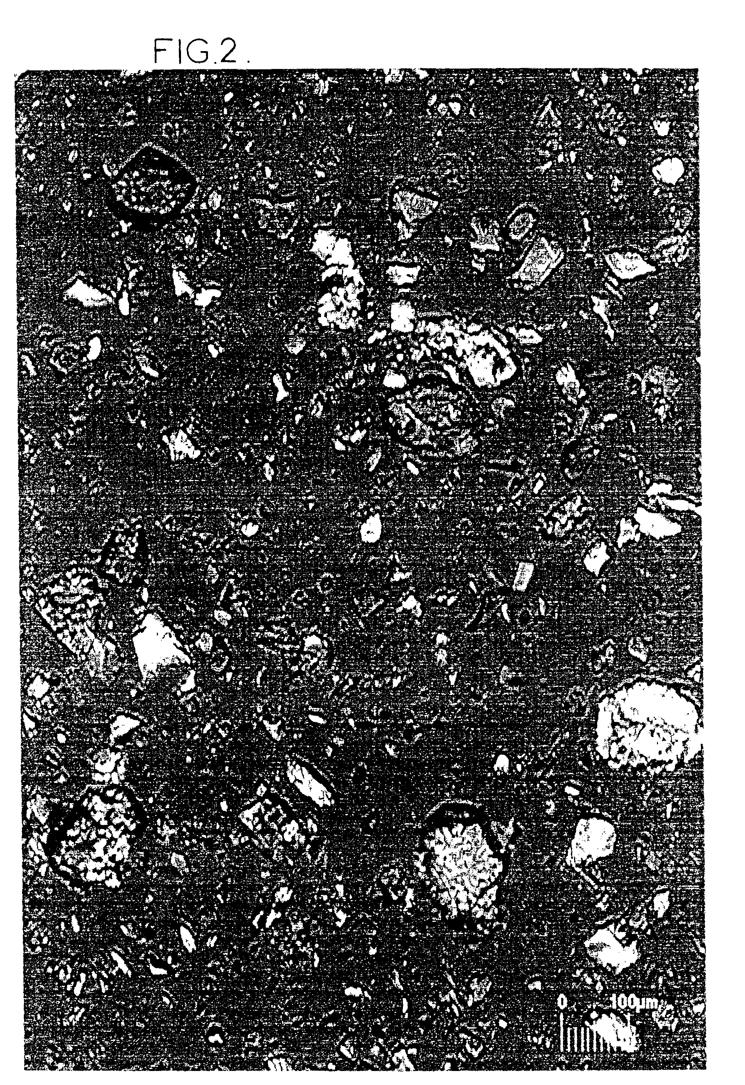

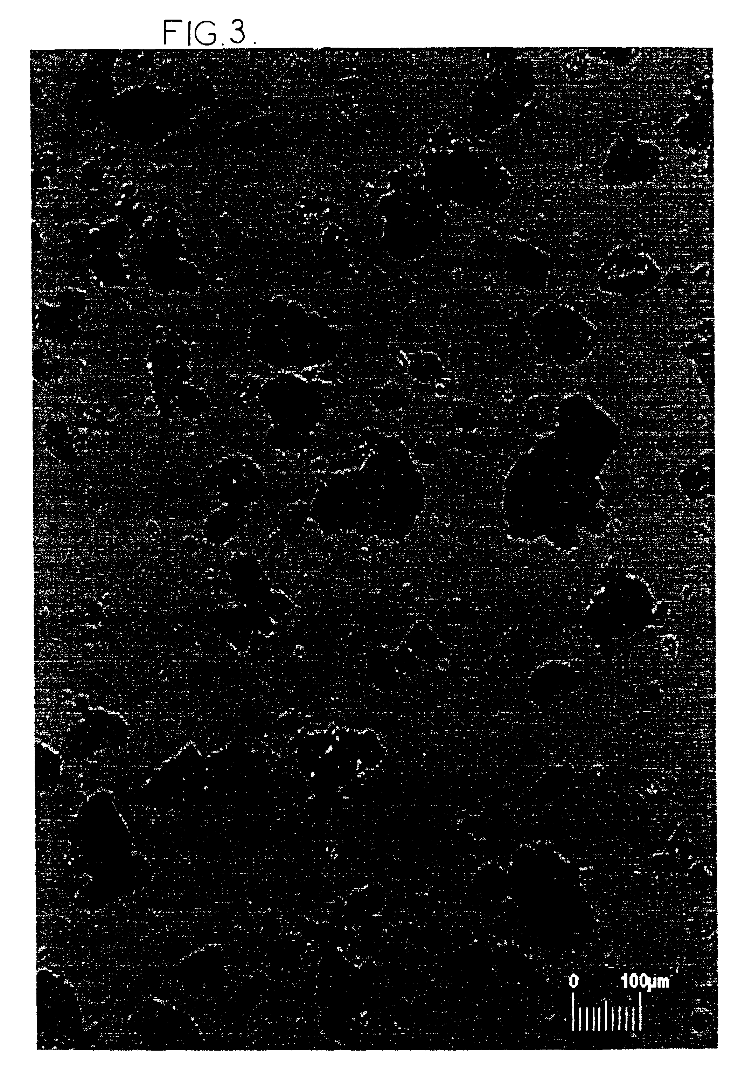

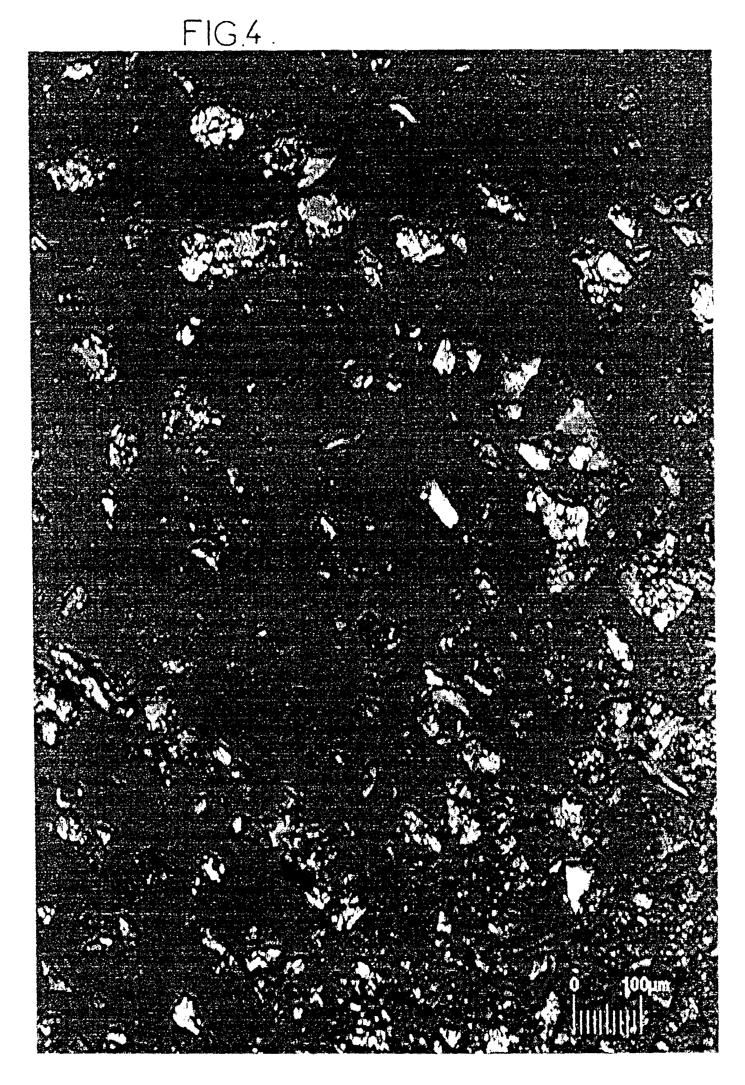

La structure des différents produits est observée au microscope optique en lumière polarisée et au microscope électronique, sur des coupes granulométriques de 0 à 100 microns. On constate par comparaison des clichés obtenus au microscope optique correspondant à la composition I1 (figure N° 1), la poudre MALTISOR® cristallisée dans l'eau (Figure N° 2), la poudre massée AMALTY® MR (figure N°3) et la poudre extrudée (Figure N°4) :
- que seule la poudre massée AMALTY® MR ne polarise pas la lumière, ce qui témoigne d'une faible cristallinité ou d'un grand désordre cristallin,
- que la composition I1 selon l'invention est essentiellement constituée de particules sphériques sans arêtes vives, ce qui la distingue très nettement des poudres cristallisées dans l'eau et extrudées.

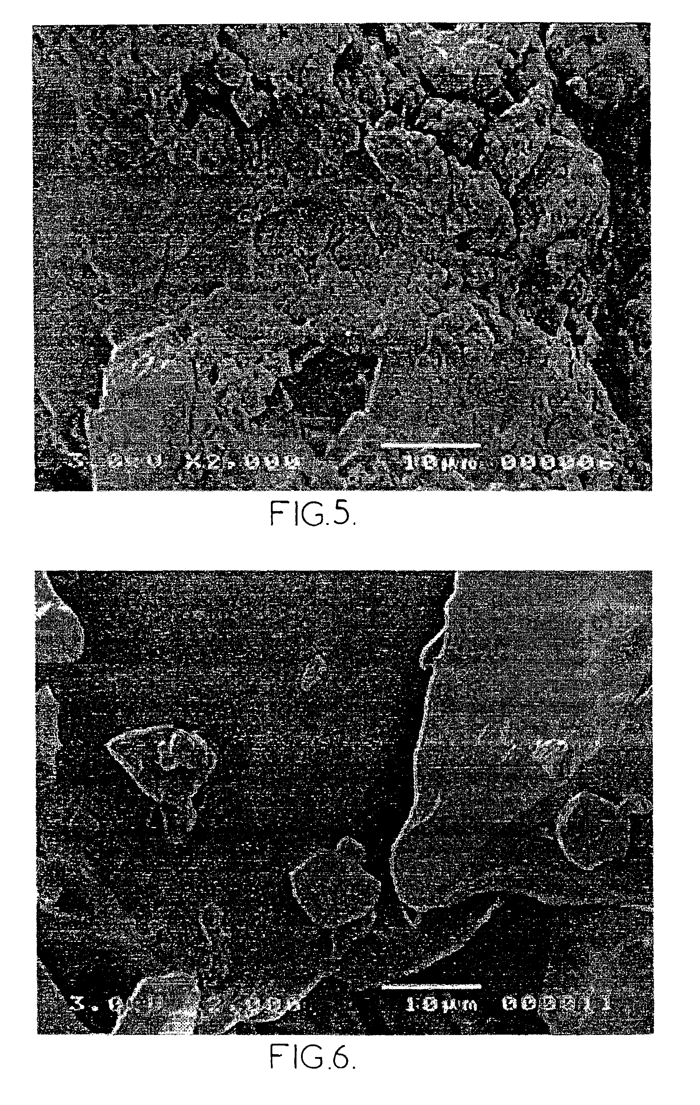

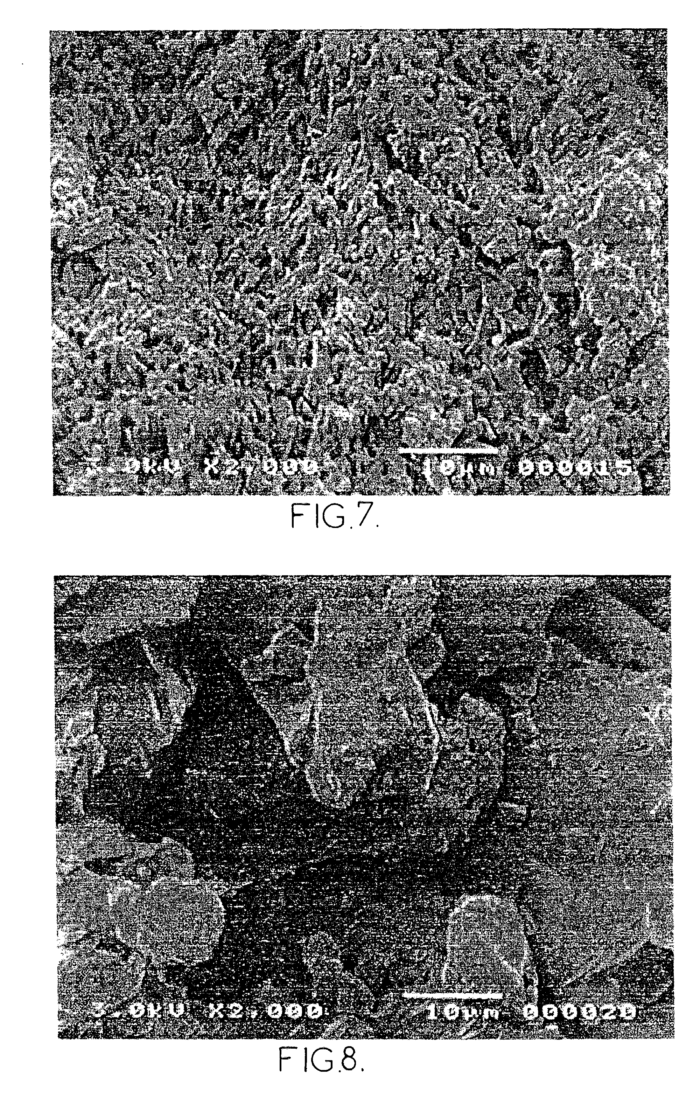

En comparant les photographies obtenues en microscopie électronique pour la composition I1 (Figure N°5), la poudre cristallisée dans l'eau (Figure N°6), la poudre massée (Figure N°7) et la poudre extrudée (Figure N°8), on constate que la composition cristalline de maltitol selon l'invention possède essentiellement une structure poreuse et alvéolée et contient des particules composées de micro-particules cristallines agglomérées entre elles. La densité de ces particules apparaît nettement moins élevée que celle des particules des produits de l'art antérieur. Ces dernières présentent en effet une structure dense et compacte, avec des surfaces de particule lisses ou hérissées, très différentes de celles retrouvées pour la composition selon l'invention.

Dans le tableau suivant sont données plusieurs caractéristiques fonctionnelles des compositions I1 et I2 selon l'invention. Contrairement aux compositions de l'art antérieur, les compositions conformes à l'invention allient avantageusement des propriétés jusqu'à présent jamais retrouvées simultanément. Elles possèdent en effet à la fois les caractéristiques d'être compressibles, de s'écouler facilement et de se dissoudre très rapidement dans l'eau.

De plus il apparaît qu'elles sont très faiblement hygroscopiques, ce qui est un avantage indéniable pour leur stockage et leur usage.

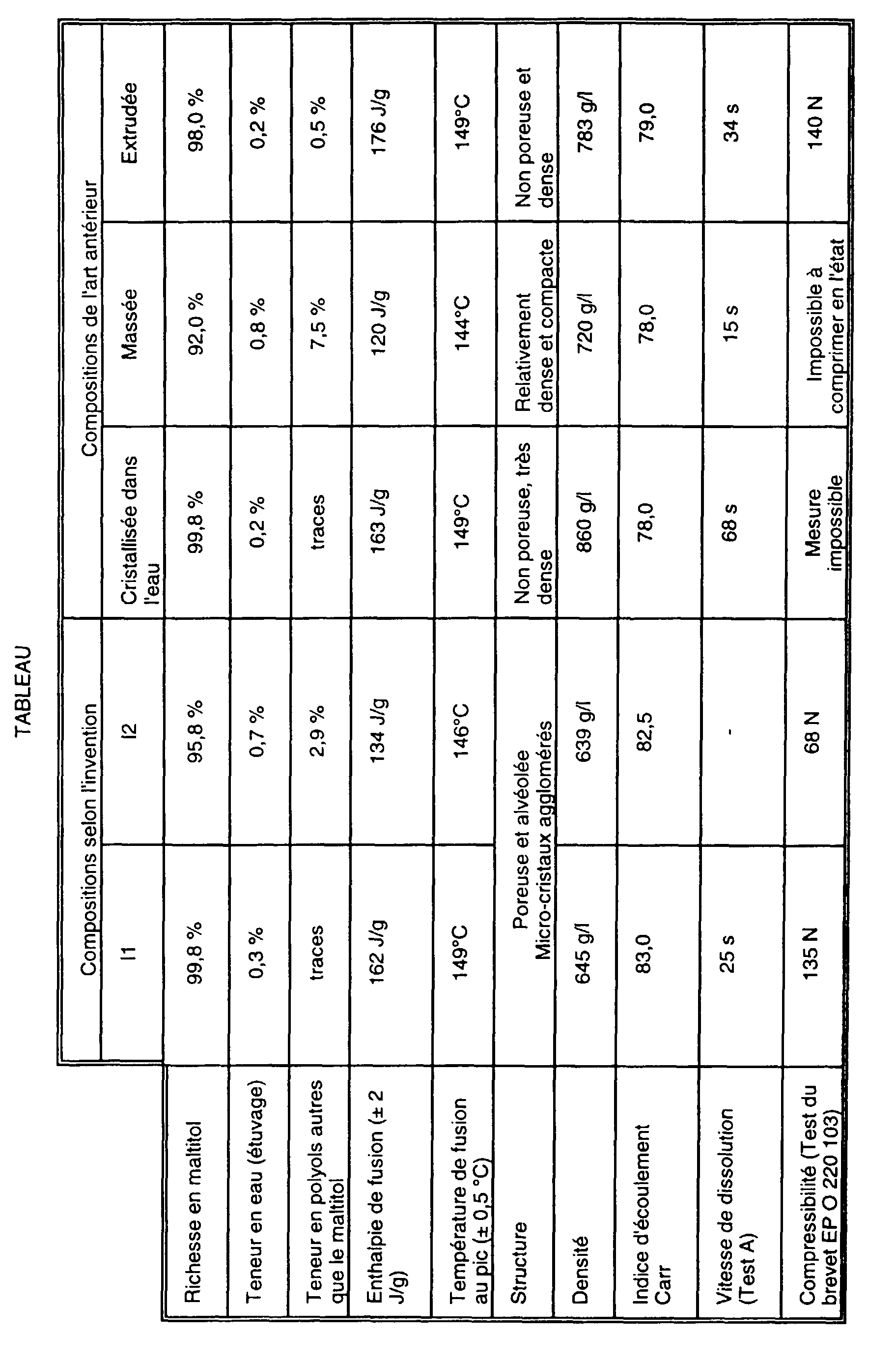

### Exemple 2 : Comparaison de compositions conformes à l'invention et des produits de l'art antérieur obtenus selon le brevet US 4,408,401.

On prépare des "total sugar" selon l'art antérieur par atomisation simple de solutions précristallisées ou masses cuites de maltitol.

Pour cela, on suit les recommandations données dans le brevet US 4,408,401, en employant des suspensions cristallines de richesse en maltitol de 98 %, contenant 25 à 60 % environ de cristaux de maltitol.

On compare les caractéristiques des "total sugar" ainsi obtenus à celles des compositions I1 et I2 selon l'invention de l'exemple 1.

On constate que les "total sugar" présentent une structure dense et une densité apparente toujours supérieure à 700 g/l, les autres caractéristiques étant également très proches d'un produit cristallisé dans l'eau. C'est ainsi que les vitesses de dissolution dans l'eau de ces "total sugar" sont voisines de 70 secondes et qu'il n'est pas possible de préparer à partir de ceux-ci des comprimés selon le test du brevet EP 0 220 103, même en augmentant les forces de compressions.

On constate que les "total sugar" de l'art antérieur ne présentent pas les caractéristiques physiques et fonctionnelles avantageuses des compositions I1 et I2 conformes à l'invention.

### Exemple 3 :

On prépare des chewing-gums sans sucre selon la formulation ci-dessous :
- gomme de base´ 247 g
- poudre de maltitol 543 g
- sirop de maltitol LYCASIN® 80/55 198 g
- arôme spearmint 12 g

On retient en tant que poudre de maltitol :
- une coupe granulométrique 200-315 microns préparée à partir de la composition I1 conforme à l'invention et donnée à l'exemple 1,
- et une coupe granulométrique 200-315 microns obtenue à partir d'une poudre de maltitol cristallisé dans l'eau MALTISORB® P200.

On compare la texture des chewing-gums sans sucre obtenus dans des conditions strictement identiques par emploi des deux poudres de maltitol ci-dessus.

On constate à la dégustation que, bien que les poudres utilisées soient particulièrement grossières, la coupe selon l'invention confère au chewing-gum une texture plus lisse et très nettement moins sableuse que la coupe de maltitol cristallisé dans l'eau.

On constate également que la dureté des échantillons de chewing-gums contenant la coupe selon l'invention est avantageusement plus grande que celle des échantillons formulés avec la coupe de l'art antérieur. Ceci est confirmé par une mesure de dureté par pénétrométrie au moyen d'un matériel de marque INSTRON®.

Cette comparaison confirme l'intérêt que présente la composition conforme à l'invention dans la formulation de chewing-gum lorsque l'on souhaite ajuster la texture.

On peut également sans inconvénient retenir la composition cristalline conforme à l'invention pour dragéifier des chewing-gums en l'utilisant sous forme pulvérulente ou de sirop.

## Revendications

1. Composition cristalline de maltitol, caractérisée en ce qu'elle présente essentiellement une structure poreuse et alvéolée, une richesse en maltitol supérieure ou égale à 92 %, et une densité apparente comprise entre 100 et 700 g/l.

2. Composition selon la revendication 1, caractérisée en ce qu'elle présente une richesse en maltitol supérieure ou égale à 95 %, de préférence supérieure ou égale à 98 % et plus préférentiellement encore égale ou supérieure à 99 %.

3. Composition selon les revendications 1 ou 2, caractérisée en ce qu'elle présente une enthalpie de fusion supérieure à 130 J/g, de préférence supérieure à 145 J/g et plus préférentiellement encore supérieure à 155 J/g.

4. Composition selon l'une quelconque des revendications 1 à 3, caractérisée en ce qu'elle contient, en poids sur matière sèche, moins de 5 % et de préférence moins de 2 % de polyols autres que le maltitol.

5. Composition selon l'une quelconque des revendications 1 à 4, caractérisée en ce qu'elle présente une densité apparente comprise entre 200 et 670 g/l, de préférence entre 300 et 650 g/l, et plus préférentiellement encore entre 400 et 650 g/l.

6. Composition selon l'une quelconque des revendications 1 à 5, caractérisée en ce qu'elle présente une note d'écoulement de Carr comprise entre 70 et 90, de préférence entre 75 et 90 et plus préférentiellement encore entre 80 et 90.

7. Composition selon l'une quelconque des revendications 1 à 6, caractérisée en ce qu'elle présente une teneur en eau inférieure à 2 %, de préférence inférieure à 1 % et plus préférentiellement encore inférieure à 0,5 %.

8. Composition selon l'une quelconque des revendications 1 à 7, caractérisée en ce qu'elle contient un ou plusieurs additifs choisis parmi les édulcorants intenses, les colorants, les parfums, les arômes, les vitamines, les minéraux, les principes actifs pharmaceutiques ou vétérinaires, les esters gras d'acides gras, les acides organiques et minéraux et leurs sels, les matières protéiques comme les protéines, les acides aminés et les enzymes.

9. Composition selon l'une quelconque des revendications 1 à 8, caractérisée en ce qu'elle présente une vitesse de dissolution dans l'eau, selon un test A, inférieure à 30 secondes, de préférence inférieure à 26 secondes et plus préférentiellement inférieure à 20 secondes.

10. Procédé d'obtention d'une composition cristalline de maltitol caractérisé en ce qu'il comporte les étapes suivantes :
- préparation d'un sirop de maltitol ayant une matière sèche d'au moins 50 % et présentant une richesse en maltitol supérieure ou égale à 92 %,
- pulvérisation fine de ce sirop sur un lit pulvérulent de particules en mouvement de maltitol cristallisé de richesse au moins égale à celle du sirop, ce lit ayant une température comprise entre 60 et 110°C; la masse du lit représentant constamment au moins 2 fois la masse du sirop pulvérisé,
- séchage du lit pulvérulent et du sirop afin d'obtenir la composition cristalline de maltitol,
- maturation éventuelle de la composition cristalline de maltitol jusqu'à ce qu'elle présente une cristallinité suffisante et de préférence une enthalpie de fusion supérieure ou égale à 130 J/g,
- recyclage éventuel partiel de la composition cristalline de maltitol afin qu'elle constitue un nouveau lit pulvérulent de maltitol cristallisé.

## Claims

1. Crystalline maltitol composition, characterized in that it essentially exhibits a porous and honeycombed structure, a maltitol concentration which is greater than or equal to 92%, and an apparent density of between 100 and 700 g/l.

2. Composition according to Claim 1, characterized in that it exhibits a maltitol concentration which is greater than or equal to 95%, preferably greater than or equal to 98%, and, still more preferably, equal to or greater than 99%.

3. Composition according to Claims 1 or 2, characterized in that it exhibits a heat of fusion which is greater than 130 J/g, preferably greater than 145 J/g and, still more preferably, greater than 155 J/g.

4. Composition according to any one of Claims 1 to 3, characterized in that it contains, in weight of dry matter, less than 5% and, preferably, less than 2%, of polyols other than maltitol.

5. Composition according to any one of Claims 1 to 4, characterized in that it exhibits an apparent density of between 200 and 670 g/l, preferably of between 300 and 650 g/l and, still more preferably, of between 400 and 650 g/l.

6. Composition according to any one of Claims 1 to 5, characterized in that it exhibits a Carr flow rating of between 70 and 90, preferably of between 75 and 90 and, still more preferably, of between 80 and 90.

7. Composition according to any one of Claims 1 to 6, characterized in that it exhibits a water content which is less than 2%, preferably less than 1% and, still more preferably, less than 0.5%.

8. Composition according to any one of Claims 1 to 7, characterized in that it contains one or more additives which are selected from among intense sweeteners, colorants, perfumes, fragrances, vitamins, minerals, pharmaceutical or veterinary active principles, fatty esters of fatty acids, organic and inorganic acids and their salts, proteinaceous substances such as proteins, amino acids and enzymes.

9. Composition according to any one of Claims 1 to 8, characterized in that it exhibits a speed of dissolution in water, according to a test A, which is less than 30 seconds, preferably less than 26 seconds and, more preferably, less than 20 seconds.

10. Process for obtaining a crystalline maltitol composition, characterized in that it comprises the following steps:
- preparing a maltitol syrup having a dry matter content of at least 50% and exhibiting a maltitol concentration which is greater than or equal to 92%,
- finely atomizing this syrup on a moving pulverulent bed of particles of crystallized maltitol at a concentration which is at least equal to that of the syrup, with this bed having a temperature of between 60 and 110°C and the mass of the bed consistently representing at least 2 times the mass of the atomized syrup,
- drying the pulverulent bed and the syrup in order to obtain the crystalline maltitol composition,
- where appropriate, maturing the crystalline maltitol composition until it exhibits a sufficient crystallinity and, preferably, a heat of fusion which is greater than or equal to 130 J/g,
- where appropriate, partially recycling the crystalline maltitol composition so that it constitutes a new pulverulent bed of crystallized maltitol.

## Patentansprüche

1. Kristalline Maltitol-Zusammensetzung, dadurch gekennzeichnet, daß sie im wesentlichen eine poröse und wabenförmige Struktur, einen Maltitol-Gehalt größer oder gleich 92% und ein Schüttvolumen von 100 bis 700 g/l aufweist.

2. Zusammensetzung nach Anspruch 1, dadurch gekennzeichnet, daß sie einen Maltitol-Gehalt größer oder gleich 95%, bevorzugt größer oder gleich 98% und noch bevorzugter größer oder gleich 99% aufweist.

3. Zusammensetzung nach Anspruch 1 oder 2, dadurch gekennzeichnet, daß sie eine Schmelzenthalpie größer 130 J/g, bevorzugt größer 145 J/g und noch bevorzugter größer 155 J/g aufweist.

4. Zusammensetzung nach einem der Ansprüche 1 bis 3, dadurch gekennzeichnet, daß sie, bezogen auf die Trockenmasse, weniger als 5 Gew.-% und bevorzugt weniger als 2 Gew.-% von Maltitol verschiedene Polyole enthält.

5. Zusammensetzung nach einem der Ansprüche 1 bis 4, dadurch gekennzeichnet, daß sie ein Schüttvolumen von 200 bis 670 g/l, bevorzugt von 300 bis 650 g/l und noch bevorzugter von 400 bis 650 g/l aufweist.

6. Zusammensetzung nach einem der Ansprüche 1 bis 5, dadurch gekennzeichnet, daß sie einen Carr'schen Rieselfaktor von 70 bis 90, bevorzugt von 75 bis 90 und noch bevorzugter von 80 bis 90 aufweist.

7. Zusammensetzung nach einem der Ansprüche 1 bis 6, dadurch gekennzeichnet, daß sie einen Wasser-Gehalt kleiner 2%, bevorzugt kleiner 1% und noch bevorzugter kleiner 0,5% aufweist.

8. Zusammensetzung nach einem der Ansprüche 1 bis 7, dadurch gekennzeichnet, daß sie einen oder mehrere unter den Süßstoffen, den Farbstoffen, den Parfums, den Aromen, den Vitaminen, den Mineralstoffen, den pharmazeutischen oder tierarzneikundlichen Wirkstoffen, den mit Fettalkoholen veresterten Fettsäuren, den organischen oder mineralischen Säuren sowie deren Salzen, den proteinartigen Substanzen, wie den Proteinen, den Aminosäuren und den Enzymen ausgewählte Zusatzstoffe enthält.

9. Zusammensetzung nach einem der Ansprüche 1 bis 8, dadurch gekennzeichnet, daß sie eine mittels eines Tests A bestimmte Lösungsgeschwindigkeit in Wasser kleiner 30 Sekunden, bevorzugt kleiner 26 Sekunden und noch bevorzugter kleiner 20 Sekunden aufweist.

10. Verfahren zur Darstellung einer kristallinen Maltitol-Zusammensetzung, dadurch gekennzeichnet, daß es folgende Schritte umfasst:
- Herstellung eines Maltitol-Sirups, der einen Anteil an Trockenmasse von mindestens 50% und einen Maltitol-Gehalt größer oder gleich 92% aufweist,
- Verarbeitung dieses Sirups zu einem feinen Pulver über einem Staubbett aus bewegten kristallisierten Maltitol-Partikeln, die einen Maltitol-Gehalt aufweisen, der größer oder gleich dem Maltitol-Gehalt des Sirups ist, wobei das Staubbett eine Temperatur von 60 bis 110 °C aufweist und die Masse des Betts stets mindestens der 2-fachen Masse des pulverisierten Sirups entspricht,
- Trocknen des Staubbetts und des Sirups unter Erhalt der kristallinen Maltitol-Zusammensetzung,
- gegebenenfalls Reifung der kristallinen Maltitol-Zusammensetzung, bis diese eine ausreichende Kristallinität und vorzugsweise eine Schmelzenthalpie größer oder gleich 130 J/g aufweist
und
- gegebenenfalls teilweise Rezyklierung der kristallinen Maltitol-Zusammensetzung zur Bildung eines neuen Staubbetts aus kristallisiertem Maltitol.
